# EUROPEAN PATENT SPECIFICATION

(11) **EP 4 493 127 B1**
(45) Date of publication and mention of the grant of the patent: **25.11.2026**
(21) Application number: 23710897.2
(22) Date of filing: 14.03.2023
(51) Int. Cl.: A61F 2/66

(54) **PROSTHESIS OR ORTHOSIS WITH VARIABLE HINGE STIFFNESS**
PROTHESE ODER ORTHESE MIT VARIABLER SCHARNIERSTEIFHEIT
PROTHÈSE OU ORTHÈSE À RIGIDITÉ CHARNIÈRE VARIABLE

(30) Priority: 14.03.2022 NL 2031270; 14.03.2022 NL 2031271; 09.01.2023 NL 2033926
(43) Date of publication of application: 22.01.2025
(73) Proprietor: Axiles Bionics BV, 1130 Brussel (BE)
(72) Inventor: CHERELLE, Pierre Philippe, 1472 VIEUX-GENAPPE (BE); GOMEZ, Felipe, 1310 LA HULPE (BE); DE MUYTER, Julien, 1000 BRUXELLES (BE); LAUWERS, Gil, 1030 SCHAERBEEK (BE)
(74) Representative: Arnold & Siedsma
(86) International application number: PCT/EP2023/056404
(87) International publication number: WO 2023/174902

(56) References cited:
- EP-A1- 3 128 958
- WO-A1-2020/039063
- GB-A- 120 874
- US-A1- 2020 214 856
- US-B2- 8 057 550

## Description

### FIELD OF INVENTION

The field of invention relates to prostheses or orthoses with variable hinge stiffness. Particular embodiments relate to matters pertaining to prostheses or orthoses for functionally assisting, enhancing, and/or replacing a limb of a human or animal subject or for augmenting a body or a part of a body of a human or animal subject.

### BACKGROUND

In existing prostheses or orthoses, reproduction of a joint motion as occurring naturally in humans and animals remains a complex technical challenge. Indeed, while walking for example, humans use a cyclic sequence of limb movements to move the body forward and maintain stance stability. This is accomplished by a process called the double pendulum. And although walking is by far the most basic and common thing in life, it involves very complex mechanisms including energy storing, transfer and return which depend on a highly complex anatomical bone, muscle, and tendon structure. The main mechanical challenge lies in accurately transitioning between the different phases of the cyclic sequence provoked by the limb movements for a user of the prosthesis or orthosis to have a more comfortable experience during use. However, each person has a cyclic sequence with different transitioning times which influence forces exerted during the joint motion. And for the same person, different responses to forces are required during the different phases of the cyclic sequence provoked by the limb movements to approximate more closely a natural motion.

In prior art solution, to address the above-mentioned problems, transitioning through the cyclic sequence has been performed through complex mechanical features in hinge joint systems; thereby increasing noticeably the production cost of prostheses or orthoses and rendering adaptability to individual users more complicated. There is thus a need for a prosthesis or orthosis comprising a hinge joint system allowing an easier mechanical adaptation to individual users, and which enables variations in responses during the different phases of the cyclic sequence provoked by the limb movements.

EP3128958A1 discloses a prosthetic foot including an attachment member, at least one first brace, at least one first flexible member, an unpowered actuator, at least one second brace, and at least one second flexible member.

### SUMMARY

The object of embodiments of the invention is to provide a prosthesis or orthosis comprising a hinge joint system allowing a simple user customizability while having a reliable mechanical structure and enabling variations in mechanical responses in different positions of the hinge joint.

According to an aspect of the invention, there is provided a prosthesis or orthosis according to claim 1. The prosthesis or orthosis comprises a hinge joint system for functionally assisting, enhancing, and/or replacing a hinge joint system of a human or animal subject. The hinge joint system comprises: a first member; a second member pivotally attached to the first member; a linkage element or an actuator connected to the first member; a flexible member connected at a first end portion to the second member and at a second end portion to the linkage element or the actuator; at least one contact stiffening element configured for being or entering in contact with the flexible member and for providing a lever action along a length of the flexible member. The at least one contact stiffening element allows to vary a hinge stiffness between the first member and the second member.

By hinge stiffness, it is meant the resistance to a torque around a pivot axis between the first member and the second member causing the first member to pivot relative to the second member.

Depending on embodiments, the at least one contact stiffening element may be a separate mechanical component affixed to the structure of the prosthesis or orthosis, or may be integrated *(i.e.* made in one piece) as a portion of the structure of the prosthesis or orthosis.

By providing at least one contact stiffening element, depending on the working direction of the flexible member, the provision of the lever action allows increasing the hinge stiffness due to the forced point of inflexion created by the at least one contact stiffening element in contact with the flexible member. By adjusting the distance at which the flexible member is with respect to an outer surface of the at least one contact stiffening element, the timing during the movements of the replaced or assisted limb at which the lever action impacts the hinge motion can be tuned. By adjusting the position of the at least one contact stiffening element with respect to the length of the flexible member, the increase in hinge stiffness can be tuned.

In the prosthesis or orthosis, the first member and the second member may correspond to portions of an articulated limb and the hinge joint system to a corresponding joint, *e.g.* an ankle joint. In the case of a foot prosthesis or orthosis, for example, load will be transmitted between the first member and the flexible member in a dorsiflexion and a plantarflexion. By transmission of the load between the first member and the flexible member, it is meant that force applied to the first member is directly converted to a load, positive or negative, exercised on the flexible member and stored as energy, and inversely, energy stored in the flexible member is directly exerted as a load to the first member translating as a force. A force applied to the second member, due to the fixation of the second member with the flexible member, will also potentially end in the transmission of load between the first member and the flexible member.

Then, due to a mounting of the flexible member within the mechanical structure of the prosthesis or orthosis in order to act on the rotation around the first axis, and to a mounting of the linkage element or actuator between the first member and the flexible member, a torque will be created between the first member and the second member. This torque may depend, amongst others, on the transmission variance of the linkage element or actuator, external forces applied to the first member, external forces applied to the second member, mechanical characteristics of the flexible member, and/or other forces applied to the flexible member by the linkage element or actuator.

Since a torque is created between the first member and the second member around the first axis, due to the flexible member being fixed to the second member on one hand and to the linkage element or actuator on the other hand, and to the second member being pivotally coupled with the first member, a rotation of the second member relative to the first member around the first axis is obtained.

When a force is applied to the second member, rotation of the flexible member with respect to the first member is achieved. This rotation may be caused by the restoring force of the flexible member which then acts on the rotation of the second member relative to the first member around the first axis. Additionally, due to the flexible member, a load applied by the subject wearing the prosthesis or orthosis may be stored and released, thereby improving the energy efficiency of the prosthesis or orthosis.

The linkage element may have a fixed length. The actuator may comprise additional active or passive elements in order to provide energy for the motion of the first member relative to the second member. Examples of active elements comprise linear or rotary motorized actuators. Examples of passive elements comprise elastic elements storing energy due to the application of a load.

Depending on embodiments, the at least one contact stiffening element may be provided on the side of the flexion and/or on the side of the extension of the flexible member. Additionally, more than one contact stiffening element may be provided to the same side at different positions along the length of the flexible member and at the same or different distances relative to the flexible member.

It is to be noted that the contact between a contact stiffening element and the flexible member may be a contact point, a contact line, or a contact surface.

Preferably, at least one of the at least one contact stiffening element is configured for entering in contact with the flexible member in a flexion or an extension of the first member relative to the second member.

Flexion and extension are movements that take place within the sagittal plane and involve anterior or posterior movements of the body or limbs. In the limbs, flexion decreases the angle between the bones (bending of the joint), while extension increases the angle and straightens the joint.

By acting on the motions taking place within the sagittal plane, the most important motions of the limbs are addressed with the at least one contact stiffening element. The skilled person will understand that the same principles may be applied to torsion motions of the limbs.

According to a preferred embodiment, at least one of the at least one contact stiffening element comprises a core made of a first material and an envelope surrounding said core made of a second material, wherein the second material has greater elasticity than the first material.

In this manner, the variation in hinge stiffness due to a contact stiffening element of the at least one contact stiffening element entering in contact with the flexible member is smoothened due to the higher elasticity of the second material. In an embodiment, the core may be made of metal, preferably steel, and the envelope may be made of silicone or rubber.

Preferably, the core has a cylindrical shape such as to provide a contact line across the width of the flexible member. The envelope may be designed with a desired profile in order to further tune the rate of variation in the hinge stiffness as the flexible member and one of the at least one contact stiffening element further enter in contact with each other.

In other embodiments, the core may have a profile with an elliptical shape, triangular shape, or rectangular shape.

The skilled person will understand that the envelope may comprise a third material with a different elasticity than the first and second material to further tune the rate of variation in the hinge stiffness during the contact.

According to an exemplary embodiment, at least one of the at least one contact stiffening element is coupled to the second member. Additionally or alternatively, at least one of the at least one contact stiffening element is coupled to the first member.

In this way, the contact between one of the at least one contact stiffening element and the flexible member may be achieved in a dynamic manner or in a static manner. Since the flexible member is connected to the second member, when one of the at least one contact stiffening element is coupled to the second member, the contact is achieved in a static manner, providing for a more accurate prediction in the variation of the hinge stiffness. Since the flexible member is connected to the linkage element or actuator pivotally connected to the first member, when one of the at least one contact stiffening element is coupled to the first member, the contact is achieved in a dynamic manner, thereby further improving a potential tunability of the non-linear variance in the hinge stiffness during a motion of the prosthesis or orthosis.

According to a typical embodiment, the flexible member comprises a blade spring. At least one of the at least one contact stiffening element is configured for providing a lever axis extending over a width of the flexible member.

Using a lever axis extending over the width of the flexible member, the lever action exerted by a contact stiffening element will be applied uniformly and in a direction opposite to the working direction. Additionally, it will prevent a torsion of the flexible member. Depending on embodiments, the blade spring may be straight or curved. Also, a ball joint may be used in addition to the blade spring at the level of the connection between the flexible member and the linkage element or actuator.

According to a preferred embodiment, at least one of the at least one contact stiffening element is mounted in a removable manner.

In this manner, replacement of the at least one of the at least one contact stiffening element is rendered easier. Also, different contact stiffening elements may be used depending on the user to further adapt the action of the flexible member to the user.

According to an exemplary embodiment, at least one of the at least one contact stiffening element is movable to a plurality of contact positions such as to provide a corresponding plurality of lever actions along the length of the flexible member.

In this way, use of the prosthesis or orthosis is more easily adapted during use for different types of activities and/or qualities of the ground. Indeed, in the example of a foot prosthesis or orthosis, depending on whether the ground is sloping up or down, depending on whether the user is sitting, walking, or running, depending on whether the ground is a forest floor or concrete floor, the response of the prosthesis or orthosis to forces applied to the flexible member will differ. With a contact stiffening element which is movable, the lever action exerted due to the contact between the contact stiffening element can be further tuned to be better adapted to the multiple varieties of environmental and activity-related conditions. In other words, the torque characteristic may be varied by changing the contact position.

In an embodiment, the contact stiffening element comprises a rotatable cam with an elongate profile, depending on the angular positioning of the cam, the lever action provided by the contact stiffening element can be rendered more or less stiff by moving a contact point between the rotatable cam and the flexible member closer or away from a middle point along the length of the flexible member.

In another embodiment, the contact stiffening element comprises a slidable pin extending perpendicularly relative to the length of the flexible member. The slidable pin is configured for sliding closer or away from a middle point along the length of the flexible member, thereby moving a contact point between the slidable pin and the flexible member.

According to a typical embodiment, the at least one movable contact stiffening element is configured for being moved from a first contact position of the plurality of contact positions to a second contact position of the plurality of contact positions by a motorized positioning actuator.

With this approach, the movable contact stiffening element may be moved more conveniently. Preferably, the movable contact stiffening element is moved from the first contact position to the second contact position when the flexible element and the movable contact stiffening element are not in contact, not to excessively burden the motorized positioning actuator.

According to a preferred embodiment, the flexible member comprises the front portion, a middle portion, and the rear portion. An average section, per unit length, of the middle portion includes less material than an average section, per unit length, of the front portion or the rear portion.

The average section of the middle portion may include less material due to an initial shaping of the flexible member, a removal of material from its sides due to machining, and/or a removal of material by piercing holes through the middle portion of the flexible member.

Preferably, the front portion has a first width, the middle portion has a second width, and the rear portion has a third width, as seen in a transverse direction perpendicular to a length direction of the flexible member, respectively. The second width is inferior to the first width and/or to the third width. In another embodiment, the front portion has a first thickness, the middle portion has a second thickness, and the rear portion has a third thickness. The second thickness is inferior to the first thickness and/or the second thickness.

Alternatively, the average section, per unit length, of the middle portion includes more material than the average section, per unit length, of the front position or the rear portion. The average section of the middle portion may include more material due to an initial shaping of the flexible member, and/or an addition of material being fixed to the middle portion of the flexible member.

In this manner, a load applied at either the front portion or the rear portion will trigger a deformation of the flexible member along its length. The front portion and the rear portion can be used as fixation points using coupling means, such as braces, clamps, bolts, and/or a chemical adhesive. In other words, the middle portion is the portion of the flexible member that is not constrained by a fixation and that is free to move and deform.

The overall flexibility characteristics of the flexible member can be tuned by tuning the geometrical shape of the middle portion. In an embodiment, the width of the flexible member may be constant over the length of the middle portion and the flexible member may be adapted from user to user by increasing or decreasing the width of the middle portion. In another embodiment, the width of the flexible member may follow a parabolic profile being at its largest at the rear portion and at the front portion. Additionally or alternatively, the thickness of the middle portion may also be adapted from user to user in the same manner as described relative to the width of the middle portion.

According to an exemplary embodiment, the front portion and the rear portion of the flexible member are each provided with at least one hole configured for cooperating with a coupling means.

In this way, the number of elements comprised in the flexible member can be reduced, thereby reducing the overall cost of the flexible member, and allowing the provision of a better mechanical reliability. The coupling means may comprise bolts cooperating with the at least one hole, preferably with a plurality of holes at each of the front portion and the rear portion.

According to a preferred embodiment, the flexible member is made in a single piece.

In this manner, the mechanical reliability of the flexible member is improved, and the manufacturing of the flexible member is rendered simpler. For example, the manufacturing of the flexible member may comprise cutting in the desired material the shape of the flexible member.

The flexible member may be made of metal, such as steel or aluminum, or composite, such as carbon or glass fiber. The flexible member may be manufactured by cutting, e.g. water-jet cutting, milling, and/or by molding.

According to an exemplary embodiment, the flexible member has a constant thickness as seen along the length direction of the flexible member. By constant thickness, it is meant that a variation of plus or minus 20% of the thickness along the length direction of the flexible member is acceptable.

Additionally, fiber layers may be added to the thickness of the flexible member at its front portion and/or its rear portion.

In this way, the overall design of the flexible member is simplified in order to make it easier to adapt the flexible member from user to user. Manufacturing is also simplified. Moreover, the mechanical reliability of the flexible member is preserved in the working directions of the flexible member, *i.e.* in directions perpendicular to the length of the flexible member.

According to a preferred embodiment, the flexible member is flat.

By flat, it is referred to the overall shape of the flexible member which, when mounted in the prothesis or orthosis, remains rectilinear in a resting position of the prosthesis or orthosis.

In this manner, manufacturing of the flexible member is simplified.

In another embodiment, the flexible member is curved or even undulated, thereby providing for modified mechanical responses of the flexible member depending on the working directions.

According to an exemplary embodiment, the flexible member comprises or consists of a plate-like element.

By plate-like element, it is referred to a manufacturing of a main component composing the flexible member. This main component originates from a sheet of material and has not been substantively modified by an additional manufacturing step. The coupling means can be provided to this main component to implement the flexible member.

In this way, manufacturing can consist in directly cutting the desired shape of the flexible member in the material of choice.

According to a preferred embodiment, the flexible member is configured for providing a hinge stiffness between the first member and the second member in a range between -40 and 40Nm/degree.

In this manner, the flexible member, when mounted in the prosthesis or orthosis, is adapted to the forces normally exerted by a user of the prosthesis or orthosis.

According to an exemplary embodiment, the flexible member comprises a cushioning member, such as a cushioning layer, configured for contacting the at least one contact stiffening element.

The cushioning member may comprise an elastic material, e.g. silicone, rubber, which enables to soften the steepness in the variation of the hinge stiffness upon contact of the flexible member with the at least one contact stiffening element. The skilled person will understand that the cushioning member may be implemented using different combinations of shapes and/or materials depending on the variation profile desired for the variation in the hinge stiffness. The cushioning member may be a homogeneous layer or may have an adapted shape and/or density distribution. Optionally, the cushioning member may be made by 3D printing.

According to a preferred embodiment, the at least one contact stiffening element is integrated as a portion of the first member or the second member.

By integrating, it is meant that the at least one contact stiffening element is made in one piece as part of another component, e.g. by casting, said another component comprised by the first member or the second member. With the at least one contact stiffening element being integrated, mechanical fatigue on the at least one contact stiffening element can be lessened. At the same time, it can serve another duty as a strengthening structural part of the first member or second member.

According to an exemplary embodiment, the second member is made of a rigid material. For example, the second member may be made of metal, such as aluminum or titanium, or a composite, such as carbon.

In this way, the modelling of the forces acting on the different elements of the prosthesis or orthosis and in direct relation with the flexible member is easier to perform. Thus, the flexible member may be tuned more accurately relative to a select user.

By "flexible", it is meant that the flexible member, when mounted in the prosthesis or orthosis, will show deformation visible by a naked eye in regular use of the prosthesis or orthosis by the user. In opposition, the second member made of the rigid material, when mounted in the prosthesis or orthosis, will not show any deformation visible by the naked eye in regular use of the prosthesis or orthosis by the user.

According to a preferred embodiment, the second member is defined by a bottom panel and/or a top panel, and two side wings. The bottom panel and/or the top panel interconnects the two side wings. The flexible member is connected to the bottom or top panel. The side wings extend on either side of the flexible member. The first member is coupled, preferably in a pivoting manner, to the two side wings.

In this manner, a structurally rigid element is defined for the second member while obtaining a relatively light second member. Additionally, each of the side wings may be provided with one or more openings in order to further reduce the weight of the second member.

In the case of a foot prosthesis or orthosis, for example, the side wings are preferably triangular-shaped to approximate a foot shape.

The skilled person will understand that features and advantages of the above-described aspects related to prosthesis and orthosis embodiments apply, *mutatis mutandis,* to the below described aspect related to prosthesis and orthosis embodiments.

According to an example, there is provided a prosthesis or orthosis comprising a hinge joint system for functionally assisting, enhancing, and/or replacing a hinge joint system of a human or animal subject. The hinge joint system comprises: a first member; a second member pivotally attached to the first member; a linkage element or an actuator connected to the first member; and a flexible member connected at a first end portion to the second member and at a second end portion to the linkage element or the actuator.

By being connected at the first end portion to the second member and at the second end portion to the linkage element or the actuator, the flexible member can act on the torque, and thus the rotation, around the first axis. The flexible member is configured for storing energy when a load is applied to it. The load applied to it may be the result of an applied torsion, elongation, tension, and/or pressure, depending on a working direction of the flexible member along which the load is applied and on a type of the flexible member.

Since the control of the rotation around the first axis in the hinge joint system depends mainly on a single mechanical piece, the flexible member, user customizability can advantageously be performed by simply tuning the characteristics of this mechanical element.

According to an aspect of the invention, there is provided a prosthesis or orthosis comprising a hinge joint system for functionally assisting, enhancing, and/or replacing a hinge joint system of a human or animal subject. The hinge joint system comprises: a first member; a second member pivotally attached to the first member; a linkage element or an actuator connected to the first member; a flexible member connected at a first end portion to the second member and at a second end portion to the linkage element or the actuator; at least one contact stiffening element configured for entering in contact with the flexible member during use of the prosthesis or orthosis and for providing a lever action along a length of the flexible member. The at least one contact stiffening element allows to vary a hinge stiffness between the first member and the second member upon entering in contact. The at least one contact stiffening element and the flexible member are unbound. In other words, the flexible member and the at least one contact stiffening element are physically distinct and are not linked by an additional mechanical component.

Taking into account a complete cycle of motion of the hinge joint system that the prosthesis or orthosis functionally assists, enhances, and/or replaces, the at least one contact stiffening element is configured for entering in contact with the flexible member at least during a portion of this motion cycle. During the portion of the motion cycle when the flexible member is in contact with the at least one contact stiffening element, the hinge stiffness of the flexible member is varied compared to the remaining portion of the motion cycle due to the additional lever action provided by the contact between the at least one contact stiffening element and the flexible member.

In some embodiments, the at least one contact stiffening element may be in contact with the flexible member in a resting state of the prosthesis or orthosis. The contact may be broken during a first part of the motion cycle and the at least one contact stiffening element and the flexible member will enter in contact again in a second part of the motion cycle.

The skilled person will understand that features and advantages of the above-described aspects related to prosthesis and orthosis embodiments apply, *mutatis mutandis,* to the below described aspect related to method embodiments.

According to an aspect of the invention, there is provided a method for adapting a prosthesis or orthosis to a user according to claim 13. The method for adapting the prosthesis or orthosis to the user comprises:
- obtaining a total weight and/or height of the user;
- providing the prosthesis or orthosis to the user, said prosthesis or orthosis comprising a hinge joint system for functionally assisting, enhancing, and/or replacing a hinge joint system of a human or animal subject, said hinge joint system comprising a first member, a second member pivotally attached to the first member, and a linkage element or an actuator connected to the first member;
- based on the obtained total weight and/or height, installing a flexible member within the prosthesis or orthosis by connecting at a first end portion thereof to the second member and at a second end portion thereof to the linkage element or the actuator.

The skilled person will understand that embodiments of the prosthesis or orthosis provided for implementing the above method may be similar to the above-described embodiments of the prosthesis or orthosis.

By doing so, the essential customization of the mechanical response from the prosthesis or orthosis can be realized in a simpler and quicker manner by installing only one adapted component of the prosthesis or orthosis, the flexible member.

According to an exemplary embodiment, the method further comprises, prior to installing the flexible member, tuning the flexible member based on the obtained total weight and/or height. According to a further embodiment, the tuning comprises, based on the obtained total weight and/or height, removing an amount of material from a middle portion of the flexible member and/or adding an amount of material to the middle portion of the flexible member.

The method further comprises, based on the obtained total weight and/or height, installing at least one contact stiffening element within the prosthesis or orthosis, said at least one contact stiffening element configured for being or entering in contact with the flexible member and for providing a lever action along a length of the flexible member.

In this way, further customization of the mechanical response from the prosthesis or orthosis is available, thereby enabling a greater comfort to the user by providing a more natural behavior of the prosthesis or orthosis.

According to a further embodiment, the installing of the at least one contact stiffening element comprises at least one of: adapting a shape of the at least one contact stiffening element, adapting a composing material of the at least one contact stiffening element, adapting a lateral positioning of the at least one contact stiffening element, adapting a number of the at least one contact stiffening element, adapting a relative distance between the at least one contact stiffening element and the flexible member in a resting position.

Optionally, the method further comprises providing the flexible member with a cushioning member configured for contacting the at least one contact stiffening element. Optionally the cushioning member may be designed or selected in function of the obtained total weight and/or height.

### BRIEF DESCRIPTION OF THE DRAWINGS

This and other aspects of the present invention will now be described in more detail, with reference to the appended drawings showing a currently preferred embodiment. Like numbers refer to like features throughout the drawings.
Figure 1 illustrates a cut side view of an exemplary embodiment of a prosthesis or orthosis;
Figure 2 depicts a top view of an exemplary embodiment of a flexible member;
Figures 3A-3C schematically show side views of another exemplary embodiment of a prosthesis or orthosis;
Figures 4A-4C schematically show side views of yet another exemplary embodiment of a prosthesis or orthosis;
Figures 5A-5B depict simple schematics of an exemplary embodiment of a foot prosthesis or orthosis and of a knee prosthesis or orthosis, respectively;
Figure 6 illustrates a perspective view of the second member of the embodiment of a prosthesis or orthosis from Figure 1;
Figure 7 depicts a top view of another exemplary embodiment of a flexible member according to the present invention;
Figure 8 depicts a side view of yet another exemplary embodiment of a flexible member according to the present invention;
Figures 9A-9B show a top perspective view of still another exemplary embodiment of a flexible member according to the present invention, and still another exemplary embodiment of a prosthesis or orthosis, respectively;
Figures 10A-10B schematically illustrate two different side views of exemplary embodiments of a foot prosthesis or orthosis with a movable contact stiffening element.

### DETAILED DESCRIPTION OF EMBODIMENTS

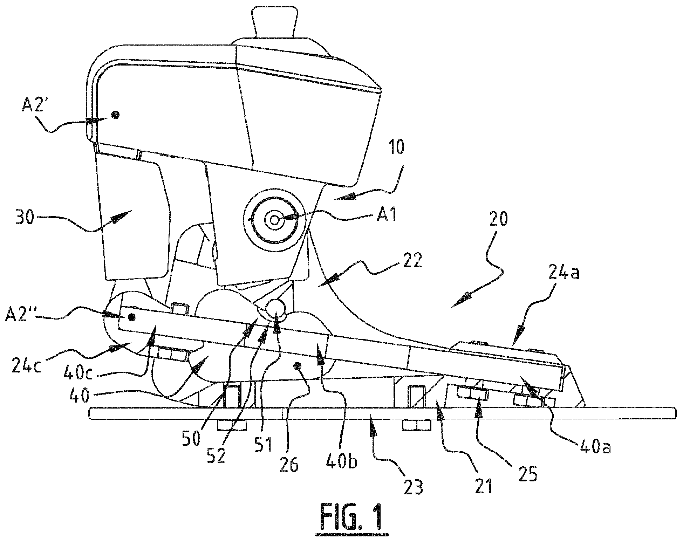

Figure 1 illustrates a cut side view of an exemplary embodiment of a prosthesis or orthosis according to the present invention. The prosthesis or orthosis comprises a hinge joint system for functionally assisting, enhancing, and/or replacing a hinge joint system of a human or animal subject. The hinge joint system comprises: a first member 10; a second member 20 pivotally attached to the first member 10; a linkage element or an actuator 30 connected to the first member 10; a flexible member 40 connected at a first end portion 40a to the second member 20 and at a second end portion 40c to the linkage element or the actuator 30.

For the sake of simplicity, embodiments related to a foot prosthesis with an ankle joint system for a human subject will be detailed in the following. The skilled person will understand, however, that the disclosed features below can be freely adapted to an animal subject or for another limb, for example.

The first member 10 may be configured to be attached to a limb of a user, *e.g.* a tibia for a transtibial foot prosthesis. The first member 10 is pivotally coupled to the second member 20 around an axis A1. In the embodiment of Figure 1, the linkage element or actuator 30 is pivotally coupled to the flexible member 40 around a second second axis A2". The second second axis A2" is substantially parallel to the first axis A1. The linkage element or actuator 30 is also pivotally coupled to the first member 10 around a first second axis A2', also substantially parallel to the first axis A1.

Briefly, when force is applied to the first member 100, it is converted to a load, positive or negative, exercised on the flexible member 40 and stored as energy, and inversely, energy stored in the flexible member 40 is directly exerted as a load to the first member 10 translating as a force. Also, due to the form factor of the flexible member 40 and to its fixation with the second member 20 in Figure 1, the storage of energy will show as a deformation of the flexible member 40 along its length.

In the embodiment of Figure 1, the linkage element 30 has a fixed length. In another embodiment, the linkage element 30 may be replaced by an actuator. The actuator may comprise additional active or passive elements in order to provide energy for the motion of the first member relative to the second member. Examples of active elements comprise linear or rotary motorized actuators. Examples of passive elements comprise elastic elements storing energy due to the application of a load. The actuator may also comprise a torque controlling mechanism such as described in patent application NL2030109.

The second member 20 may be defined by a bottom and/or top panel, a bottom panel 21 in Figure 1, and two side wings 22, preferably made of a rigid material such as metal or composite, such as carbon. In the embodiment of Figure 1, the bottom panel 21 interconnects the two side wings 22. The second member 20 depicted in Figure 1 comprises a sole element 23, preferably made of composite material, such as carbon, or aluminum. The sole element 23 may be configured for being covered with a layer of rubber (not shown) or an overall foot cover (not shown) configured for being in contact with the ground during a gait cycle. The flexible member 40 is fixed to the second member 20, to the bottom panel 21 in the embodiment of Figure 1. The fixation of the flexible member 40 may be completed by a brace 24a and a coupling means 25, such as bolts. A front portion 40a of the flexible member 40 is fastened between the brace 24a and a top surface of the bottom panel 21. More specifically, a compartment defined by a recess in a bottom surface of the bottom panel 21 and by the sole element 23 is used in the embodiment of Figure 1 to hide the bolts heads. Alternatively or additionally, the coupling means may include brackets, clamps, bolts, braces, and/or a chemical adhesive.

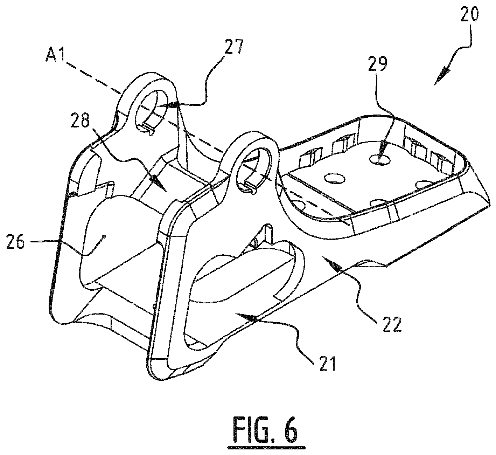

The side wings 22 may extend on either side of the flexible member 40, substantially perpendicularly relative to the bottom panel 21. The first member 10 is pivotally connected to the two side wings 22 around the first axis A1. In the embodiment of Figure 1, the side wings 22 are substantially triangular-shaped to approximate a foot shape and are provided with one opening 26 each. A more detailed illustration of the second member 20 is found in Figure 6.

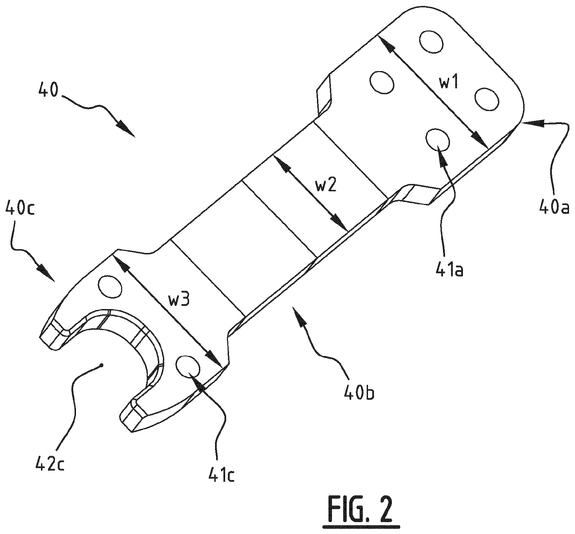

The flexible member 40 comprises the front portion 40a, a middle portion 40b, and a rear portion 40c. Embodiments of the flexible member 40 will be better described with reference to Figure 2. The rear portion 40c is used as a fixation point with the linkage element 30 or actuator around the second second axis A2". Similarly as with the front portion 40a, in the embodiment of Figure 1, a brace 24c pivotally connected to the linkage element 30 is used in combination with a coupling means, e.g. bolts, to affix the rear portion 40c. As depicted in Figure 1, a thickness of the flexible member 40 is constant along its length.

The prosthesis or orthosis may further comprise at least one contact stiffening element 50 configured for being or entering in contact with the flexible member 40, a single contact stiffening element 50 configured for entering in contact in Figure 1, and for providing a lever action along the length of the flexible member 40 such as to vary a hinge stiffness between the first member 10 and the second member 20. Preferably, at least one of the at least one contact stiffening element 50 is configured for entering in contact with the flexible member 40 in a flexion or an extension of the first member 10 relative to the second member 20, in a flexion of the first member 10 relative to the second member 20 in the embodiment of Figure 1.

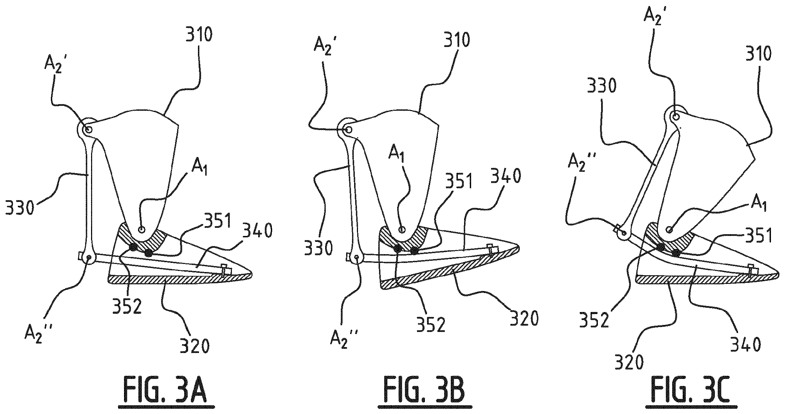

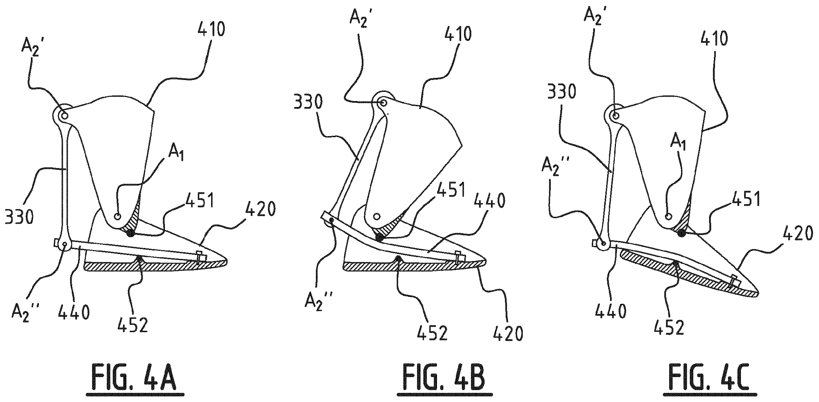

Depending on embodiments, the at least one contact stiffening element 50 may be provided on the side of the flexion and/or on the side of the extension of the flexible member 40. Additionally, more than one contact stiffening element 50 may be provided to the same side at different positions along the length of the flexible member 40 and at the same or different distance relative to the flexible member 40. The contact between a contact stiffening element of the at least one contact stiffening element 50 and the flexible member 40 may be a contact point, a contact line, or a contact surface. Additional embodiments of the at least one contact stiffening element 50 provided to a prosthesis or orthosis will be found with respect to Figures 3A-3C and Figures 4A-4C.

In the embodiment of Figure 1, the contact stiffening element 50 comprises a core 51 made of a first material and an envelope 52 surrounding said core 51 made of a second material. The second material has greater elasticity than the first material. For example, the core 51 may be made of metal, and the envelope 52 may be made of silicone or rubber. The core 51 is cylindrical-shaped such as to provide a contact line across the width of the flexible member 40. The envelope 52 has a curved profile such as to increase a contact surface with flexible member 40 as the flexion of the first member 10 with respect to the second member increases. In other embodiments, the core may have a ball shape, or may have a profile with an elliptical shape, triangular shape, or rectangular shape. Additionally, the skilled person will understand that the envelope may comprise a third material with a different elasticity than the first and second material to further tune the rate of variation in the hinge stiffness during the contact.

Further, in the embodiment of Figure 1, the contact stiffening element 50 is coupled to the second member 20, forward of the first axis A1. The contact stiffening element 50 may be coupled in a removable manner to the second member 20. In a neutral (or resting) position of the prosthesis or orthosis, the contact stiffening element 50 is positioned at a distance from a surface of the flexible member 40. The contact stiffening element 50 is provided facing a rear half of the flexible member 40. In another embodiment at least one of the at least contact stiffening element is coupled to the first member 10.

As depicted in Figure 1, during the flexion of the first member 10 relative to the second member 20, the flexible member 40 will enter in contact with the contact stiffening element 50. Upon entering in contact, a hinge stiffness around the first axis A1 will increase since a lever arm created by the flexible member 40 will decrease in length due to the forced point of inflexion achieved by the contact stiffening element 50.

The skilled person will understand that numerous variable hinge stiffness schemes may be implemented by using one or more contact stiffening elements at different positions relative to the flexible member.

Figure 2 depict a top view of an exemplary embodiment of a flexible member according to the present invention. The flexible member 40 comprises a front portion 40a, a middle portion 40b, and a rear portion 40c. The front portion 40a has a first width w1, the middle portion 40b has a second width w2, and the rear portion 40c has a third width w3.

An average section, per unit length, of the middle portion 40b includes less material than an average section, per unit length, of the front portion 40a or the rear portion 40c. More particularly, in the embodiment of Figure 2, the second width w2 is inferior to the first width w1 and to the third width w3. The flexible member 40 has a constant thickness as seen along the length direction of the flexible member 40. The second width w2 of the flexible member 40 may be constant over the length of the middle portion 40b and the flexible member 40 may be adapted from user to user by increasing or decreasing the second width w2. Also, the flexible member 40 is flat and consists of a plate-like element. Preferably, the plate-like element forms a blade spring.

In another embodiment, the flexible member may be curved. In yet another embodiment, the width of the flexible member may follow a parabolic profile being at its largest at the rear portion and at the front portion. Additionally or alternatively, the thickness of the middle portion may also be adapted from user to user in the same manner as described relative to the width of the middle portion.

Both of the rear portion 40c and the front portion 40a may be provided in the embodiment of Figure 2 with a plurality of holes 41a, 41c, two holes 41c at the rear portion 40c and four holes 41a at the front portion 40a, configured for cooperating with a coupling means (not shown), e.g. a corresponding plurality of bolts. A U-shaped opening 42c in the rear portion 40c may allow for an extremity of a linkage element or actuator (not shown) to be arranged therein in order to improve compacity.

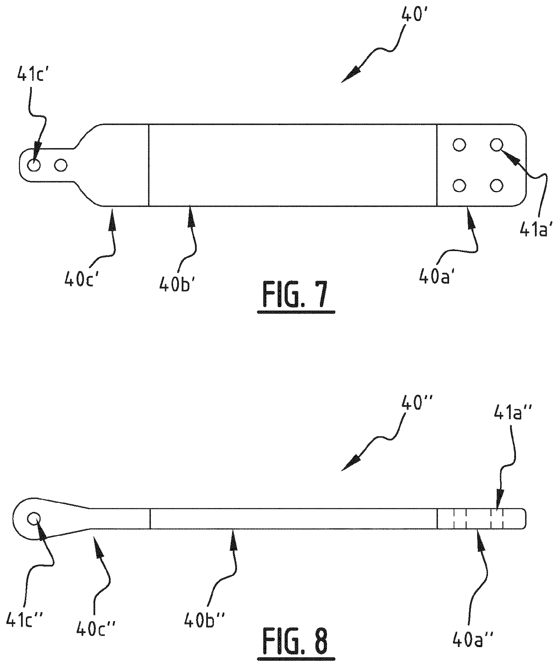

Figures 7 and 8 depict a top view and a side view, respectively, of other exemplary embodiments of a flexible member according to the present invention.

In the embodiment of Figure 7, the flexible member 40' comprises a front portion 40a', a middle portion 40b', and a rear portion 40c'. In the embodiment of Figure 8, the flexible member 40" comprises a front portion 40a", a middle portion 40b", and a rear portion 40c". In the embodiments of Figs.7-8, an average section, per unit length, of the middle portion includes more material than an average section, per unit length, of the front portion or the rear portion.

The flexible member 40', 40" has a constant thickness as seen along the length direction of the flexible member 40', 40". A width of the flexible member 40', 40" may be constant over the length of the middle portion 40b', 40b" and the flexible member 40', 40" may be adapted from user to user by increasing or decreasing the width of the middle portion 40b', 40b". Also, the flexible member 40', 40" is flat and consists of a plate-like element. Preferably, the plate-like element forms a blade spring.

In the embodiment of Figure 7, both of the rear portion 40c' and the front portion 40a' may be provided with a plurality of holes 41a', 41c', two holes 41c' at the rear portion 40c' and four holes 41a' at the front portion 40a', configured for cooperating with a coupling means (not shown), *e.g.* a corresponding plurality of bolts. The rear portion 40c' may have a tapered profile when seen from the top. The front portion 40a' may have a constant width along its length. In an alternative embodiment, both the front portion 40a' and the rear portion 40c' may have a tapered profile when seen from the top and may be provided with two holes each. In yet another embodiment, the front portion 40a' may have a tapered profile when seen from the top and the rear portion 40c' may have a constant width along its length.

In the embodiment of Figure 8, the front portion 40a" may be identical to the one depicted in Figure 7. The rear portion 40c" may be tapered in a manner similar to the one depicted in Figure 7. Instead of being provided with a plurality of holes, the rear portion 40c" may be provided with a side hole 41c" in a thicker section of the rear portion 40c". The side hole 41c" may be suitable for being provided with an axle (not shown) allowing rotation of the flexible member 40" relative to the linkage element or actuator (not shown).

Figures 3A-3C schematically show side views of another exemplary embodiment of a prosthesis or orthosis according to the present invention. In the three figures, the prosthesis or orthosis comprises a hinge joint system for functionally assisting, enhancing, and/or replacing a hinge joint system of a human or animal subject. The hinge joint system comprises: a first member 310; a second member 320 pivotally attached to the first member 310; a linkage element or an actuator 330 connected to the first member 310; a flexible member 340 connected at a first end portion to the second member 320 and at a second end portion to the linkage element or the actuator 330.

The first member 310 may be configured to be attached to a limb of a user. The first member 310 is pivotally coupled to the second member 320 around an axis A1. In the embodiments of Figures 3A-3C, the linkage element or actuator 330 is pivotally coupled to the flexible member 340 around a second second axis A2". The second second axis A2" is substantially parallel to the first axis A1. The linkage element or actuator 330 is also pivotally coupled to the first member 310 around a first second axis A2'.

The prosthesis or orthosis further comprises, in the embodiments of Figures 3A-3C, a first and a second contact stiffening elements 351, 352 configured for being or entering in contact with the flexible member 340 and for providing a lever action along the length of the flexible member 340 such as to vary a hinge stiffness between the first member 310 and the second member 320. The first contact stiffening element 351 and the second contact stiffening element 352 are coupled to the second member 320, above the flexible member 340.

In a neutral (or resting) position of the prosthesis or orthosis in the embodiment of Figure 3A, the first and the second contact stiffening elements 351, 352 are at a distance from the flexible member 340. The second contact stiffening element 352 is provided at a further distance from the flexible member 340 than the first contact stiffening element 352. The first contact stiffening element 351 is provided closer to the front of the second member 320 than the second contact stiffening element 352.

In the embodiment of Figure 3B, a flexion motion between the first member 310 and the second member 320 brings the flexible member 340 in contact with the first contact stiffening element 351. Upon being brought in contact with the flexible member 340, the first contact stiffening element 351 induces an upward deformation of the flexible member 340. The hinge stiffness around the first axis A1 increases.

In the embodiment of Figure 3C, the flexion motion between the first member 310 and the second member 320 has proceeded until the flexible member 340 reached contact with the second contact stiffening element 352. Upon being brought in contact with the flexible member 340, both the first contact stiffening element 351 and the second contact stiffening element 352 induce an upward deformation of the flexible member 340. The hinge stiffness around the first axis A1 increases further.

Figures 4A-4C schematically show side views of yet another exemplary embodiment of a prosthesis or orthosis according to the present invention. In the three figures, the prosthesis or orthosis comprises a hinge joint system for functionally assisting, enhancing, and/or replacing a hinge joint system of a human or animal subject. The hinge joint system comprises: a first member 410; a second member 420 pivotally attached to the first member 410; a linkage element or an actuator 430 connected to the first member 410; a flexible member 440 connected at a first end portion to the second member 420 and at a second end portion to the linkage element or the actuator 430.

The first member 410 may be configured to be attached to a limb of a user. The first member 410 is pivotally coupled to the second member 420 around an axis A1. In the embodiments of Figures 4A-4C, the linkage element or actuator 430 is pivotally coupled to the flexible member 440 around a second second axis A2". The second second axis A2" is substantially parallel to the first axis A1. The linkage element or actuator 430 is also pivotally coupled to the first member 410 around a first second axis A2'.

The prosthesis or orthosis further comprises, in the embodiments of Figures 4A-4C, a first and a second contact stiffening elements 451, 452 configured for being or entering in contact with the flexible member 440 and for providing a lever action along the length of the flexible member 440 such as to vary a hinge stiffness between the first member 410 and the second member 420. The first contact stiffening element 451 is coupled to the first member 410 above the flexible member 440, and the second contact stiffening element 452 is coupled to the second member 420 below the flexible member 440.

In a neutral (or resting) position of the prosthesis or orthosis in the embodiment of Figure 3A, the first contact stiffening element 451 is at a distance from the flexible member 440, and the second contact stiffening element 452 is in contact with the flexible member 440. The second contact stiffening element 452 is provided closer to the front of the second member 420 than the first contact stiffening element 451. In an alternative embodiment, the first contact stiffening element 451 is in contact with the flexible member 440, and the second contact stiffening element 452 is at a distance from the flexible member 440

In the embodiment of Figure 4B, a flexion motion between the first member 410 and the second member 420 brings the flexible member 440 in contact with the first contact stiffening element 451. Upon being brought in contact with the flexible member 440, the first contact stiffening element 451 induces an upward deformation of the flexible member 440. The hinge stiffness around the first axis A1 increases.

In the embodiment of Figure 4C, an extension motion between the first member 410 and the second member 420 has been performed while the flexible member 440 was already in contact with the second contact stiffening element 452 from the neutral position. Upon performing this extension motion in contact with the flexible member 440, the second contact stiffening element 352 induces a downward deformation of the flexible member 440. The hinge stiffness around the first axis A1 increases with respect to the neutral position.

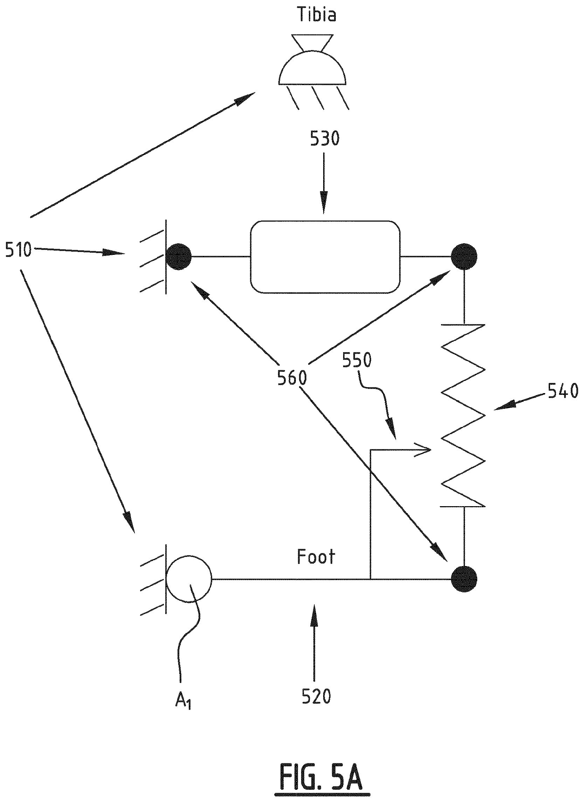

Figures 5A-5B depict simple schematics of an exemplary embodiment of a foot prosthesis or orthosis and of a knee prosthesis or orthosis, respectively, according to the present invention. When implementing a hinge joint system, the mechanical basics require to have a pivot coupling around a main axis A1 between a first member 510 and a second member 520.

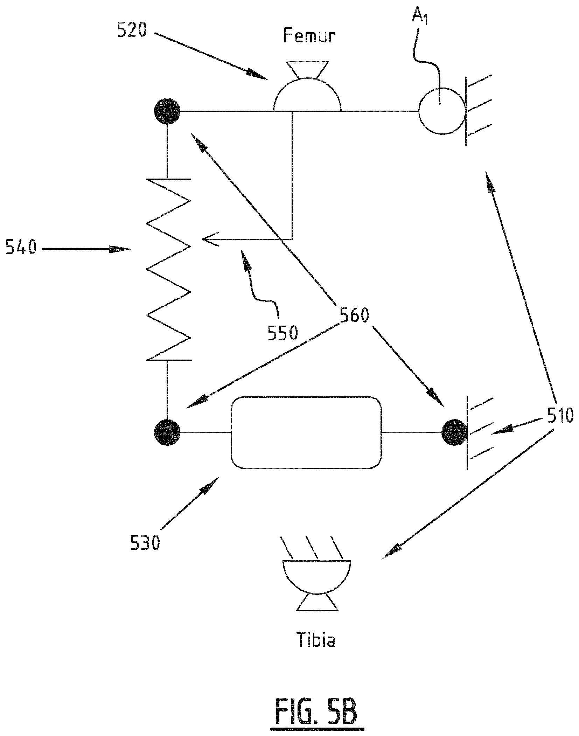

In the embodiment of Figure 5A, the first member 510 is provided with an attachment to a tibia, the second member 520 corresponds to the foot, and the main axis A1 represents an ankle joint. In the embodiment of Figure 5B, the first member 510 is provided with an attachment to a femur, the second member 520 is provided with an attachment to a tibia, and the main axis A1 represents a knee joint.

In addition, in parallel to this pivot coupling around the main axis A1, a linkage element or actuator 530 coupled to a flexible member 540 are provided in series between the first member 210 and the second member 520. The combination of the linkage element or actuator 530 coupled to the flexible member 540 allows to modulate the torque around the main axis A1 between the first member 510 and the second member 520. In principle, between each of the elements of the hinge joint system, *i.e.* between the first member 510 and the linkage element or actuator 530, between the linkage element or actuator 530 and the flexible member 540, and/or between the flexible member 540 and the second member 520, a connection 560 may be a pivot coupling, not necessarily around an axis substantially parallel to A1, or may be a fixed coupling. In Figure 5A the flexible member 540 is provided closer to the foot, and in Figure 5B the flexible member 540 is provided closer to the femur.

Also, at least one contact stiffening element 550 is comprised by the hinge joint system. The at least one contact stiffening element 550 is configured for being or entering in contact with the flexible member 540 and for providing a lever action along a length of the flexible member 540 such as to vary a hinge stiffness between the first member 510 and the second member 520. In both embodiments of Figures 5A-5B, the at least one contact stiffening element 550 is coupled to the second member 520.

Figure 6 illustrates a perspective view of the second member of the embodiment of a prosthesis or orthosis from Figure 1 according to the invention. In the embodiment of Figure 6, the second member 20 is defined by the bottom panel 21 interconnecting the two side wings 22, preferably made of a rigid material such as metal or composite, such as carbon. Additionally, the side wings 22 are substantially triangular-shaped to approximate a foot shape and are provided with one opening 26 each. In the embodiment of Figure 6, the two side wings 22 prolong and join at the front of the second member 20, thereby improving the structural integrity of the second member.

One or more fixation holes 29 are configured for cooperating with the coupling means (not shown) such as to enable the fixation of the flexible member (not shown) to the second member 20. The one or more fixation holes (29) may be provided in a front part of the bottom panel 21. In a rear part of the bottom panel 21, an opening may be made in order to lighten the second member 20.

The fixation of the flexible member may be completed by a brace and the coupling means, such as bolts. A recess formed in a bottom surface of the bottom panel 21 may be used to hide salient parts of the coupling, for example the bolts heads. Alternatively or additionally, the coupling means may include brackets, clamps, bolts, braces, and/or a chemical adhesive.

The side wings 22 may extend on either side of the bottom panel 21, substantially perpendicularly relative to the bottom panel 21. A pair of axle slots 27 may be provided to an upper part of the side wings 22. The pair of axle slots 27 may be used to couple the first member (not shown) to the two side wings 22 in a pivoting manner. It will define the first axis A1. Below the pair of axle slots 27, a central structuring element 28 may be provided to increase the structural integrity of the second member 20. The central structuring element 28 may be located above the flexible member, when mounted in the prosthesis or orthosis. The at least one contact stiffening element (not shown) may be fixed to the central structuring element 28.

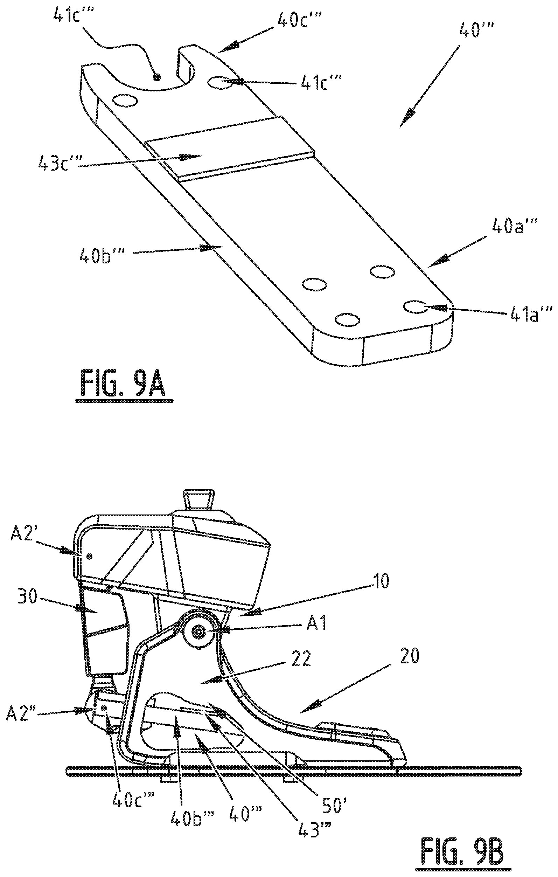

Figures 9A-9B show a top perspective view of still another exemplary embodiment of a flexible member according to the present invention, and still another exemplary embodiment of a prosthesis or orthosis, respectively, according to the present invention.

Figure 9A depicts a top perspective view of another exemplary embodiment of a flexible member according to the present invention. The flexible member 40‴ comprises a front portion 40a‴, a middle portion 40b'", and a rear portion 40c'". An average section, per unit length, of the middle portion 40b‴includes less material than an average section, per unit length, of the front portion 40a‴ or the rear portion 40c'". More particularly, in the embodiment of Figure 9A, the thickness of the middle portion 40b‴ is inferior to the thicknesses of the front portion 40a‴ and the rear portion 40c'". The flexible member 40‴ has a constant width as seen along the length direction of the flexible member 40‴.

Both of the rear portion 40c‴ and the front portion 40a‴ may be provided in the embodiment of Figure 9A with a plurality of holes 41a‴, 41c'", two holes 41c‴ at the rear portion 40c‴ and four holes 41a‴ at the front portion 40a‴, configured for cooperating with a coupling means (not shown), e.g. a corresponding plurality of bolts. A U-shaped opening 42c‴ in the rear portion 40c‴ may allow for an extremity of a linkage element or actuator (not shown) to be arranged therein in order to improve compacity.

Additionally, in the embodiment of Figure 9A, the flexible member 40‴ comprises a cushioning layer 43‴. The cushioning layer 43‴ is configured for contacting the at least one contact stiffening element, referred to as 50' in the embodiment of Figure 9B. The cushioning layer 43'" comprises rubber in the embodiment of Fig.9A, and is made of a single layer of constant thickness provided to a top surface of the middle portion 40b'". In other embodiments, the cushioning layer 43‴ may comprise at least two layers of different materials. Additionally or alternatively, a shape and/or thickness and/or density distribution of the cushioning layer 43‴ may be tailored to provide different cushioning characteristics during the contacting of the flexible member 40‴ with the at least one contact stiffening element 50'.

In the embodiment of Fig.9B, a foot prosthesis very similar to the embodiment of Fig.1 is depicted, using the flexible member of Fig.9A, and with a different at least one contact stiffening element 50'.

The prosthesis or orthosis comprises a hinge joint system for functionally assisting, enhancing, and/or replacing a hinge joint system of a human or animal subject. The hinge joint system comprises: a first member 10; a second member 20 pivotally attached to the first member 10; a linkage element or an actuator 30 connected to the first member 10; a flexible member 40‴ connected at a first end portion (not shown in Fig.9B) to the second member 20 and at a second end portion 40c‴ to the linkage element or the actuator 30. The at least one contact stiffening element 50' is configured for entering in contact with the flexible member 40‴ during use of the prosthesis or orthosis and for providing a lever action along a length of the flexible member 40‴. The at least one contact stiffening element 50' allows to vary a hinge stiffness between the first member 10 and the second member 20 upon entering in contact. The at least one contact stiffening element 50' and the flexible member 40‴ are unbound in the embodiment of Figure 9A.

The first member 10 may be configured to be attached to a limb of a user, *e.g.* a tibia for a transtibial foot prosthesis. The first member 10 is pivotally coupled to the second member 20 around an axis A1. In the embodiment of Figure 9B, the linkage element or actuator 30 is pivotally coupled to the flexible member 40‴ around a second second axis A2". The second second axis A2" is substantially parallel to the first axis A1. The linkage element or actuator 30 is also pivotally coupled to the first member 10 around a first second axis A2', also substantially parallel to the first axis A1.

In the embodiment of Fig.9B, the at least one contact stiffening element 50' is integrated to the second member 20. More particularly, the at least one contact stiffening element 50' extends transversally between two side wings 22 of the second member, thereby improving the structural integrity of the second member 20. In another embodiment, the at least one contact stiffening element 50' is affixed to the second member 20, between the two side wings 22 of the second member for example.

The at least one contact stiffening element 50' protrudes downward, in direction of the flexible member 40‴. Facing the at least one contact stiffening element 50', the flexible member 40‴ is provided with the cushioning layer 43‴. The at least one contact stiffening element 50' and the cushioning layer 43‴ of the flexible member 40‴ will enter in contact during an extension motion of the prosthesis.

In another embodiment, the cushioning layer 43‴ may comprise one or more elastic materials, e.g. silicone, rubber, stacked and/or arranged in one or more layers. Additionally or alternatively, the cushioning layer 43‴ may be implemented using different combinations of shapes depending on the variation profile desired for the variation in the hinge stiffness.

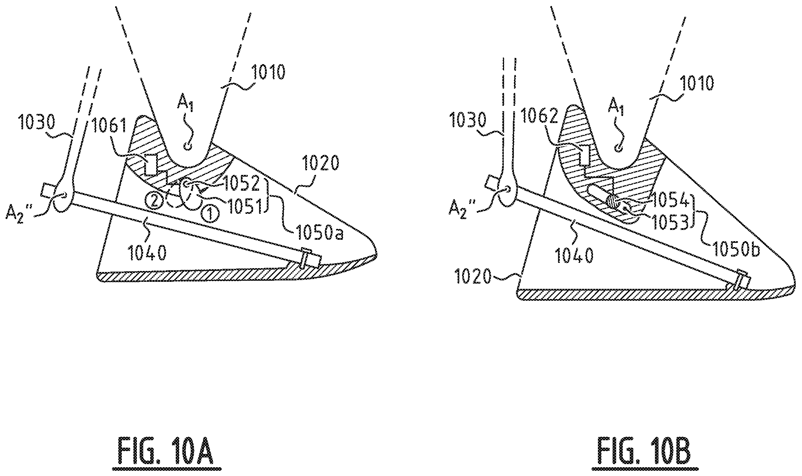

Figures 10A-10B schematically illustrate two different side views of exemplary embodiments of a foot prosthesis or orthosis with a movable contact stiffening element according to the present invention.

In the two figures, Fig.10A and Fig.10B, the prosthesis or orthosis comprises a hinge joint system for functionally assisting, enhancing, and/or replacing a hinge joint system of a human or animal subject. The hinge joint system comprises: a first member 1010; a second member 1020 pivotally attached to the first member 1010; a linkage element or an actuator 1030 connected to the first member 1010; a flexible member 1040 connected at a first end portion to the second member 1020 and at a second end portion to the linkage element or the actuator 1030.

The first member 1010 may be configured to be attached to a limb of a user. The first member 1010 is pivotally coupled to the second member 1020 around an axis A1. In the embodiments of Figures 10A-10C, the linkage element or actuator 1030 is pivotally coupled to the flexible member 1040 around a second second axis A2". The second second axis A2" is substantially parallel to the first axis A1. The linkage element or actuator 1030 is also pivotally coupled to the first member 1010 around a first second axis (not shown).

The prosthesis or orthosis further comprises, in the embodiment of Fig.10A a first movable contact stiffening element 1050a, and in the embodiment of Fig.10B a second movable contact stiffening element 1050b. Both first and second movable contact stiffening elements 1050a, 1050b are configured for being or entering in contact with the flexible member 1040 and for providing a lever action along the length of the flexible member 1040 such as to vary a hinge stiffness between the first member 1010 and the second member 1020. More particularly, both the first and second movable contact stiffening elements 1050a, 1050b are configured for being movable to a plurality of contact positions such as to provide a corresponding plurality of lever actions along the length of the flexible member 1040.

In the embodiment of Fig.10A, the first movable contact stiffening element 1050a is coupled to the second member 1020 above the flexible member 1040. The first movable contact stiffening element 1050a may comprise a rotatable cam 1051 with an elongate profile and may be coupled with a pivot 1052 to the first member 1010. The pivot 1052 may have a rotation axis substantially parallel to the axis A1. Depending on the angular positioning of the rotatable cam 1051, the lever action provided by the first contact stiffening element 1050a can be rendered more or less stiff by moving a contact point between the rotatable cam 1051 and the flexible member 1040 closer or away from a middle point along the length of the flexible member 1040. More particularly, in position 1 as illustrated in Fig.10A, the flexible member 1040 will have a stiffer response than in position 2.

In the embodiment of Fig.10B, the second movable contact stiffening element 1050b is also coupled to the second member 1020 above the flexible member 1040. The second movable contact stiffening element 1050b may comprise a slidable pin 1054 extending perpendicularly relative to the length of the flexible member 1040. The slidable pin 1054 is configured for sliding closer or away from a middle point along the length of the flexible member 1040, thereby moving a contact point between the slidable pin 1054 and the flexible member 1040. To guide the slidable pin 1054, an elongate guiding means 1053, *e.g.* an elongate guiding hole, may be provided to the second member. In the embodiment of Fig.10B the elongate guiding means 1053 extends substantially parallel to the length of the flexible member 1040 in a resting position.

Additionally, the first and second movable contact stiffening elements 1050a, 1050b may be configured for being moved from a first contact position of the plurality of contact positions to a second contact position of the plurality of contact positions by a motorized positioning actuator 1061, 1062. To move the rotatable cam 1051 of the first movable contact stiffening element 1050a, a rotary motorized actuator 1061 may be employed. And to move the slidable pin 1054 of the second movable contact stiffening element 1050b, a linear motorized actuator 1062 may be employed. Preferably, the first and second movable contact stiffening elements 1050a, 1050b are moved from the first contact position to the second contact position when the flexible element 1040 and the first and second movable contact stiffening elements 1050a, 1050b are not in contact, respectively. The skilled person will understand that, in other embodiments, one can also implement a movable contact stiffening configured for being moved from the first contact position of the plurality of contact positions to the second contact position of the plurality of contact positions using both rotational and translational motions.

Whilst the principles of the invention have been set out above in connection with specific embodiments, it is to be understood that this description is merely made by way of example and not as a limitation of the scope of protection which is determined by the appended claims.

## Claims

1. A prosthesis or orthosis comprising a hinge joint system for functionally assisting, enhancing, and/or replacing a hinge joint system of a human or animal subject, said hinge joint system comprising:
- a first member (10);
- a second member (20) being pivotally attached to the first member (10);
- a linkage element or an actuator (30) connected to the first member (10);
- a flexible member (40) connecting the second member (20) and the linkage element or the actuator (30);
**characterized in that** the hinge joint system further comprises at least one contact stiffening element (50), said at least one contact stiffening element (50) being unbound and physically distinct from the flexible member (40), wherein the at least one contact stiffening element (50) is configured for being or entering in contact with the flexible member (40) during use,
and for providing a lever action along a length of the flexible member (40).

2. The prosthesis or orthosis of claim 1, wherein at least one of the at least one contact stiffening element (50) is configured for being or entering in contact with the flexible member (40) in a flexion or an extension of the first member (10) relative to the second member (20).

3. The prosthesis or orthosis of claim 1 or 2, wherein at least one of the at least one contact stiffening element (50) comprises a core (51) made of a first material and an envelope (52) surrounding said core made of a second material, wherein the second material has greater elasticity than the first material.

4. The prosthesis or orthosis of any one of the previous claims, wherein at least one of the at least one contact stiffening element (50) is coupled to the second member (20); and/or wherein at least one of the at least one contact stiffening element (50) is coupled to the first member (10).

5. The prosthesis or orthosis of any one of the previous claims, wherein the flexible member (40) comprises a blade spring, and wherein at least one of the at least one contact stiffening element is configured for providing a lever axis extending over a width of the flexible member (40); and/or wherein at least one of the at least one contact stiffening element (50) is mounted in a removable manner.

6. The prosthesis or orthosis of any one of the previous claims, wherein at least one of the at least one contact stiffening element (50) is movable to a plurality of contact positions such as to provide a corresponding plurality of lever actions along the length of the flexible member (40); and, optionally, wherein the at least one movable contact stiffening element (50) is configured for being moved from a first contact position of the plurality of contact positions to a second contact position of the plurality of contact positions by a motorized positioning actuator.

7. The prosthesis or orthosis of any one of the previous claims, wherein the flexible member (40) is connected at a front portion (40a) to the second member (20) and at a rear portion (40c) to the linkage element or the actuator (30).

8. The prosthesis or orthosis of claim 7, wherein the flexible member (40) comprises the front portion (40a), a middle portion (40b), and the rear portion (40c); and
wherein an average section, per unit length, of the middle portion (40b) includes less material than an average section, per unit length, of the front portion (40a) or the rear portion (40c), or includes more material than the average section, per unit length, of the front position (40a', 40a") or the rear portion (40c', 40c"); and/or
wherein the front portion (40a) and the rear portion (40c) of the flexible member (40) are each provided with at least one hole configured for cooperating with a coupling means.

9. The prosthesis or orthosis of claim 7 or 8, wherein the flexible member (40) is made in a single piece; wherein the flexible member (40) has preferably a constant thickness as seen along the length direction of the flexible member (40); and, optionally, wherein the flexible member (40) is flat.

10. The prosthesis or orthosis of any one of the previous claims, wherein the flexible member (40) comprises or consists of a plate-like element.

11. The prosthesis or orthosis of any one of the previous claims, wherein the second member (20) is made of a rigid material;
wherein the second member (20) is defined by a bottom panel (21) and/or a top panel, and two side wings (22), said bottom panel (21) and/or top panel interconnecting the two side wings (22);
wherein the flexible member (40) is connected to the bottom or top panel (21);
wherein the side wings (22) extend on either side of the flexible member (40); and
wherein the first member (10) is coupled to the two side wings (22).

12. The prosthesis or orthosis of any one of the previous claims, wherein the flexible member further comprises a cushioning member configured for contacting the at least one contact stiffening element, said cushioning member being made preferably of an elastomeric material.

13. A method for adapting a prosthesis or orthosis to a user, comprising:
- obtaining a total weight and/or height of the user;
- providing the prosthesis or orthosis according to any of claims 1-12 to the user, said prosthesis or orthosis comprising a hinge joint system for functionally assisting, enhancing, and/or replacing a hinge joint system of a human or animal subject, said hinge joint system comprising a first member, a second member pivotally attached to the first member, and a linkage element or an actuator connected to the first member;
- based on the obtained total weight and/or height, installing a flexible member within the prosthesis or orthosis by connecting at a first end portion thereof to the second member and at a second end portion thereof to the linkage element or the actuator; and **characterized in that** the method further comprises:
- based on the obtained total weight and/or height, installing at least one contact stiffening element within the prosthesis or orthosis, said at least one contact stiffening element (50) being unbound and physically distinct from the flexible member (40), and said at least one contact stiffening element (50) being configured for being or entering in contact with the flexible member and for providing a lever action along a length of the flexible member.

14. The method of claim 13, further comprising, prior to installing the flexible member, tuning the flexible member based on the obtained total weight and/or height; and, wherein the tuning preferably comprises, based on the obtained total weight and/or height, removing an amount of material from a middle portion of the flexible member and/or adding an amount of material to the middle portion of the flexible member.

15. The method of claim 13 or 14, wherein the installing of the at least one contact stiffening element comprises at least one of: adapting a shape of the at least one contact stiffening element, adapting a composing material of the at least one contact stiffening element, adapting a lateral positioning of the at least one contact stiffening element, adapting a number of the at least one contact stiffening element, adapting a relative distance between the at least one contact stiffening element and the flexible member in a resting position; and, wherein the method preferably further comprises providing the flexible member with a cushioning member configured for contacting the at least one contact stiffening element, said cushioning member being optionally selected or designed in function of the obtained total weight and/or height.

## Patentansprüche

1. Prothese oder Orthese, umfassend ein Winkelgelenksystem zum funktionellen Unterstützen, Verbessern und/oder Ersetzen eines Winkelgelenksystems eines menschlichen oder tierischen Subjekts, das Winkelgelenksystem umfassend:
- ein erstes Glied (10);
- ein zweites Glied (20), das an dem ersten Glied (10) schwenkbar befestigt ist;
- ein Verlinkungselement oder einen Aktuator (30), die mit dem ersten Glied (10) verbunden sind;
- ein flexibles Glied (40), das das zweite Glied (20) und das Verlinkungselement oder den Aktuator (30) verbindet;
**dadurch gekennzeichnet, dass** das Winkelgelenksystem ferner mindestens ein Kontaktversteifungselement (50) umfasst, wobei das mindestens eine Kontaktversteifungselement (50) von dem flexiblen Glied (40) ungebunden und physisch unterschiedlich ist, wobei das mindestens eine Kontaktversteifungselement (50) konfiguriert ist, um mit dem flexiblen Glied (40) während der Verwendung in Kontakt zu sein oder zu treten, und zum Bereitstellen einer Hebelwirkung entlang einer Länge des flexiblen Glieds
(40).

2. Prothese oder Orthese nach Anspruch 1, wobei mindestens eines des mindestens einen Kontaktversteifungselements (50) konfiguriert ist, um mit dem flexiblen Glied (40) bei einer Flexion oder einer Extension des ersten Glieds (10) relativ zu dem zweiten Glied (20) in Kontakt zu sein oder zu treten.

3. Prothese oder Orthese nach Anspruch 1 oder 2, wobei mindestens eines des mindestens einen Kontaktversteifungselements (50) einen Kern (51), der aus einem ersten Material gefertigt ist, und eine den Kern umgebende Hülle (52), die aus einem zweiten Material gefertigt ist, umfasst, wobei das zweite Material eine größere Elastizität als das erste Material aufweist.

4. Prothese oder Orthese nach einem der vorstehenden Ansprüche, wobei mindestens eines des mindestens einen Kontaktversteifungselements (50) an das zweite Glied (20) gekoppelt ist; und/oder wobei mindestens eines des mindestens einen Kontaktversteifungselements (50) an das erste Glied (10) gekoppelt ist.

5. Prothese oder Orthese nach einem der vorstehenden Ansprüche, wobei das flexible Glied (40) eine Blattfeder umfasst, und wobei mindestens eines des mindestens einen Kontaktversteifungselements zum Bereitstellen einer Hebelachse konfiguriert ist, die sich über eine Breite des flexiblen Glieds (40) erstreckt; und/oder wobei mindestens eines des mindestens einen Kontaktversteifungselements (50) auf entfernbare Weise montiert ist.

6. Prothese oder Orthese nach einem der vorstehenden Ansprüche, wobei mindestens eines des mindestens einen Kontaktversteifungselements (50) in eine Vielzahl von Kontaktpositionen bewegbar ist, um so eine entsprechende Vielzahl von Hebelwirkungen entlang der Länge des flexiblen Glieds (40) bereitzustellen; und, optional, wobei das mindestens eine bewegbare Kontaktversteifungselement (50) konfiguriert ist, um von einer ersten Kontaktposition der Vielzahl von Kontaktpositionen in eine zweite Kontaktposition der Vielzahl von Kontaktpositionen durch einen motorisierten Positionierungsaktuator bewegt zu werden.

7. Prothese oder Orthese nach einem der vorstehenden Ansprüche, wobei das flexible Glied (40) an einem vorderen Abschnitt (40a) mit dem zweiten Glied (20) und an einem hinteren Abschnitt (40c) mit dem Verlinkungselement oder dem Aktuator (30) verbunden ist.

8. Prothese oder Orthese nach Anspruch 7, wobei das flexible Glied (40) den vorderen Abschnitt (40a), einen mittleren Abschnitt (40b) und den hinteren Abschnitt (40c) umfasst; und
wobei ein durchschnittlicher Teil, pro Längeneinheit, des mittleren Abschnitts (40b) weniger Material einschließt als ein durchschnittlicher Teil pro Längeneinheit des vorderen Abschnitts (40a) oder des hinteren Abschnitts (40c), oder mehr Material einschließt als der durchschnittliche Teil pro Längeneinheit der vorderen Position (40a', 40a") oder des hinteren Abschnitts (40c', 40c"); und/oder
wobei der vordere Abschnitt (40a) und der hintere Abschnitt (40c) des flexiblen Glieds (40) jeweils mit mindestens einem Loch versehend sind, das zum Zusammenwirken mit einem Kopplungsmittel konfiguriert ist.

9. Prothese oder Orthese nach Anspruch 7 oder 8, wobei das flexible Glied (40) als ein einziges Stück gefertigt ist; wobei das flexible Glied (40) vorzugsweise eine konstante Dicke entlang der Längsrichtung des flexiblen Glieds (40) gesehen aufweist; und, optional, wobei das flexible Glied (40) flach ist.

10. Prothese oder Orthese nach einem der vorstehenden Ansprüche, wobei das flexible Glied (40) ein plattenartiges Element umfasst oder daraus besteht.

11. Prothese oder Orthese nach einem der vorstehenden Ansprüche, wobei das zweite Glied (20) aus einem starren Material gefertigt ist;
wobei das zweite Glied (20) durch eine Bodenplatte (21) und/oder eine Deckplatte und zwei Seitenflügel (22) definiert ist, wobei die Bodenplatte (21) und/oder die Deckplatte die zwei Seitenflügel (22) miteinander verbinden;
wobei das flexible Glied (40) mit der Boden- oder der Deckplatte (21) verbunden ist;
wobei sich die Seitenflügel (22) auf beiden Seiten des flexiblen Glieds (40) erstrecken; und
wobei das erste Glied (10) an die zwei Seitenflügel (22) gekoppelt ist.

12. Prothese oder Orthese nach einem der vorstehenden Ansprüche, wobei das flexible Glied ferner ein Dämpfungsglied, das zum Kontaktieren des mindestens einen Kontaktversteifungselement konfiguriert ist, umfasst, wobei das Dämpfungsglied vorzugsweise aus einem elastomeren Material gefertigt ist.

13. Verfahren zum Anpassen einer Prothese oder Orthese an einen Benutzer, umfassend:
- Erhalten eines Gesamtgewichts und/oder einer Größe des Benutzers;
- Bereitstellen der Prothese oder Orthese nach einem der Ansprüche 1 bis 12 für den Benutzer, die Prothese oder Orthese umfassend ein Winkelgelenksystem zum funktionellen Unterstützen, Verbessern und/oder Ersetzen eines Winkelgelenksystems eines menschlichen oder tierischen Subjekts, das Winkelgelenksystem umfassend ein erstes Glied, ein zweites Glied, das an dem ersten Glied schwenkbar angebracht ist, und ein Verlinkungselement oder einen Aktuator, die mit dem ersten Glied verbunden sind;
- basierend auf dem erhaltenen Gesamtgewicht und/oder der Höhe, Einbauen eines flexiblen Glieds innerhalb der Prothese oder Orthese durch Verbinden an einem ersten Endabschnitt desselben mit dem zweiten Glied und an einem zweiten Endabschnitt desselben mit dem Verlinkungselement oder dem Aktuator; und
**dadurch gekennzeichnet, dass** das Verfahren ferner umfasst:
- basierend auf dem erhaltenen Gesamtgewicht und/oder der Höhe, Einbauen mindestens eines Kontaktversteifungselements innerhalb der Prothese oder Orthese, wobei das mindestens eine Kontaktversteifungselement (50) von dem flexiblen Glied (40) ungebunden und physisch unterschiedlich ist, und wobei das mindestens eine Kontaktversteifungselement (50) konfiguriert ist, um mit dem flexiblen Glied in Kontakt zu sein oder zu treten, und zum Bereitstellen einer Hebelwirkung entlang einer Länge des flexiblen Glieds.

14. Verfahren nach Anspruch 13, ferner umfassend, vor dem Einbauen des flexiblen Glieds, Abstimmen des flexiblen Glieds basierend auf dem erhaltenen Gesamtgewicht und/oder der Höhe; und wobei das Abstimmen vorzugsweise, basierend auf dem erhaltenen Gesamtgewicht und/oder der Höhe, das Entfernen einer Materialmenge von einem mittleren Abschnitt des flexiblen Glieds und/oder ein Hinzufügen einer Materialmenge zu dem mittleren Abschnitt des flexiblen Glieds umfasst.

15. Verfahren nach Anspruch 13 oder 14, wobei das Einbauen des mindestens einen Kontaktversteifungselements mindestens eines umfasst von: Anpassen einer Form des mindestens einen Kontaktversteifungselements, Anpassen eines Zusammensetzungsmaterials des mindestens einen Kontaktversteifungselements, Anpassen einer lateralen Positionierung des mindestens einen Kontaktversteifungselements, Anpassen einer Anzahl des mindestens einen Kontaktversteifungselements, Anpassen eines relativen Abstands zwischen dem mindestens einen Kontaktversteifungselement und dem flexiblen Glied in einer Ruheposition; und wobei das Verfahren vorzugsweise ferner das Versehen des flexiblen Glieds mit einem Dämpfungsglied, das zum Kontaktieren des mindestens einen Kontaktversteifungselements konfiguriert ist, umfasst, wobei das Dämpfungsglied optional in Abhängigkeit des erhaltenen Gesamtgewichts und/oder der Höhe ausgewählt oder gestaltet ist.

## Revendications

1. Prothèse ou orthèse comprenant un système d'articulation à charnière permettant d'assister, d'améliorer, et/ou de remplacer de manière fonctionnelle un système d'articulation à charnière d'un sujet humain ou animal, ledit système d'articulation à charnière comprenant :
- un premier élément (10) ;
- un second élément (20) étant fixé de manière pivotante au premier élément (10) ;
- un élément de liaison ou un actionneur (30) relié au premier élément (10) ;
- un élément souple (40) reliant le second élément (20) et l'élément de liaison ou l'actionneur (30) ;
**caractérisée en ce que** le système d'articulation à charnière comprend en outre au moins un élément de raidissement de contact (50), ledit au moins un élément de raidissement de contact (50) étant non lié et physiquement distinct de l'élément souple (40), dans laquelle l'au moins un élément de raidissement de contact (50) est conçu pour être ou entrer en contact avec l'élément souple (40) pendant l'utilisation, et pour fournir un effet de levier sur une longueur de l'élément souple
(40).

2. Prothèse ou orthèse selon la revendication 1, dans laquelle au moins l'un de l'au moins un élément de raidissement de contact (50) est conçu pour être ou entrer en contact avec l'élément souple (40) lors d'une flexion ou d'une extension du premier élément (10) par rapport au second élément (20).

3. Prothèse ou orthèse selon la revendication 1 ou 2, dans laquelle au moins l'un de l'au moins un élément de raidissement de contact (50) comprend une partie centrale (51) constituée d'un premier matériau et une enveloppe (52) entourant ladite partie centrale constituée d'un second matériau, dans laquelle le second matériau présente une plus grande élasticité que le premier matériau.

4. Prothèse ou orthèse selon l'une quelconque des revendications précédentes, dans laquelle au moins l'un de l'au moins un élément de raidissement de contact (50) est accouplé au second élément (20) ; et/ou dans laquelle au moins l'un de l'au moins un élément de raidissement de contact (50) est accouplé au premier élément (10).

5. Prothèse ou orthèse selon l'une quelconque des revendications précédentes, dans laquelle l'élément souple (40) comprend un ressort à lame, et dans laquelle au moins l'un de l'au moins un élément de raidissement de contact est conçu pour fournir un axe de levier s'étendant sur une largeur de l'élément souple (40) ; et/ou dans laquelle au moins l'un de l'au moins un élément de raidissement de contact (50) est monté de manière amovible.

6. Prothèse ou orthèse selon l'une quelconque des revendications précédentes, dans laquelle au moins l'un de l'au moins un élément de raidissement de contact (50) est mobile vers une pluralité de positions de contact de manière à fournir une pluralité correspondante d'actions de levier sur la longueur de l'élément souple (40) ; et, éventuellement, dans laquelle l'au moins un élément de raidissement de contact mobile (50) est conçu pour être déplacé d'une première position de contact de la pluralité de positions de contact à une seconde position de contact de la pluralité de positions de contact par un actionneur de positionnement motorisé.

7. Prothèse ou orthèse selon l'une quelconque des revendications précédentes, dans laquelle l'élément flexible (40) est relié au niveau d'une partie avant (40a) au second élément (20) et au niveau d'une partie arrière (40c) à l'élément de liaison ou à l'actionneur (30).

8. Prothèse ou orthèse selon la revendication 7, dans laquelle l'élément souple (40) comprend la partie avant (40a), une partie médiane (40b) et la partie arrière (40c) ; et
dans laquelle une section moyenne, par unité de longueur, de la partie médiane (40b) comporte moins de matériau qu'une section moyenne, par unité de longueur, de la partie avant (40a) ou de la partie arrière (40c), ou comporte plus de matériau que la section moyenne, par unité de longueur, de la position avant (40a', 40a") ou de la partie arrière (40c', 40c") ; et/ou
dans laquelle la partie avant (40a) et la partie arrière (40c) de l'élément souple (40) sont chacune pourvues d'au moins un trou conçu pour coopérer avec un moyen d'accouplement.

9. Prothèse ou orthèse selon la revendication 7 ou 8, dans laquelle l'élément souple (40) est réalisé en une seule pièce ; dans laquelle l'élément souple (40) présente de préférence une épaisseur constante vue le long de la direction de la longueur de l'élément souple (40) ; et, éventuellement, dans laquelle l'élément souple (40) est plat.

10. Prothèse ou orthèse selon l'une quelconque des revendications précédentes, dans laquelle l'élément souple (40) comprend ou est constitué d'un élément en forme de plaque.

11. Prothèse ou orthèse selon l'une quelconque des revendications précédentes, dans laquelle le second élément (20) est constitué d'un matériau rigide ;
dans laquelle le second élément (20) est défini par un panneau inférieur (21) et/ou un panneau supérieur, et deux ailes latérales (22), ledit panneau inférieur (21) et/ou panneau supérieur interconnectant les deux ailes latérales (22) ;
dans laquelle l'élément souple (40) est relié au panneau inférieur ou supérieur (21) ;
dans laquelle les ailes latérales (22) s'étendent de part et d'autre de l'élément souple (40) ; et dans laquelle le premier élément (10) est accouplé aux deux ailes latérales (22).

12. Prothèse ou orthèse selon l'une quelconque des revendications précédentes, dans laquelle l'élément souple comprend en outre un élément d'amortissement conçu pour venir en contact avec l'au moins un élément de raidissement de contact, ledit élément d'amortissement étant de préférence constitué d'un matériau élastomère.

13. Procédé permettant d'adapter une prothèse ou une orthèse à un utilisateur, comprenant :
- l'obtention d'un poids total et/ou d'une taille de l'utilisateur ;
- la fourniture de la prothèse ou de l'orthèse selon l'une quelconque des revendications 1 à 12 à l'utilisateur, ladite prothèse ou orthèse comprenant un système d'articulation à charnière pour assister, améliorer et/ou remplacer de manière fonctionnelle un système d'articulation à charnière d'un sujet humain ou animal, ledit système d'articulation à charnière comprenant un premier élément, un second élément fixé de manière pivotante au premier élément, et un élément de liaison ou un actionneur relié au premier élément ;
- sur la base du poids total et/ou de la taille obtenus, l'installation d'un élément souple à l'intérieur de la prothèse ou de l'orthèse en le reliant, au niveau d'une première partie d' extrémité de celui-ci, au second élément et, au niveau d'une seconde partie d' extrémité de celui-ci, à l'élément de liaison ou à l'actionneur ; et **caractérisé en ce que** le procédé comprend en outre :
- sur la base du poids total et/ou de la taille obtenus, l'installation d'au moins un élément de raidissement de contact à l'intérieur de la prothèse ou de l'orthèse, ledit au moins un élément de raidissement de contact (50) étant non lié et physiquement distinct de l'élément souple (40), et ledit au moins un élément de raidissement de contact (50) étant conçu pour être ou entrer en contact avec l'élément souple et pour fournir une action de levier sur une longueur de l'élément souple.

14. Procédé selon la revendication 13, comprenant en outre, avant d'installer l'élément souple, le réglage de l'élément souple sur la base du poids total et/ou de la hauteur obtenus ; et, dans lequel le réglage comprend de préférence, sur la base du poids total et/ou de la hauteur obtenus, le retrait d'une quantité de matériau d'une partie médiane de l'élément souple et/ou l'ajout d'une quantité de matériau à la partie médiane de l'élément souple.

15. Procédé selon la revendication 13 ou 14, dans lequel l'installation de l'au moins un élément de raidissement de contact comprend au moins l'un parmi : l'adaptation d'une forme de l'au moins un élément de raidissement de contact, l'adaptation d'un matériau constitutif de l'au moins un élément de raidissement de contact, l'adaptation d'un positionnement latéral de l'au moins un élément de raidissement de contact, l'adaptation d'un nombre de l'au moins un élément de raidissement de contact, l'adaptation d'une distance relative entre l'au moins un élément de raidissement de contact et l'élément souple dans une position de repos ; et, dans lequel le procédé comprend en outre de préférence la fourniture de l'élément souple d'un élément d'amortissement conçu pour venir en contact avec l'au moins un élément de raidissement de contact, ledit élément d'amortissement étant éventuellement choisi ou conçu en fonction du poids total et/ou de la hauteur obtenus.
